# Europäisches Patentamt
## European Patent Office
## Office européen des brevets

(19)

(11) Veröffentlichungsnummer: **0 355 635**

**A1**

(12) ## EUROPÄISCHE PATENTANMELDUNG

(21) Anmeldenummer: 89114949.4

(51) Int. Cl.⁴: **A61L 15/07**

(22) Anmeldetag: 12.08.89

(30) Priorität: 18.08.88 US 233597

(43) Veröffentlichungstag der Anmeldung:
28.02.90 Patentblatt 90/09

(84) Benannte Vertragsstaaten:
AT BE CH DE ES FR GB GR IT LI LU NL SE

(71) Anmelder: **CARAPACE INC.**
**P.O. Box 470040**
**Tulsa, OK 74147(US)**

(72) Erfinder: **Clark, James L.**
**9211 East 59th Street**
**Tulsa, OK 74145(US)**
Erfinder: **Ozsahin, Ali**
**4364 S. 109th Avenue Apt. 605**
**Tulsa, OK 74145(US)**

(74) Vertreter: **Klöpsch, Gerald, Dr.-Ing.**
**An Gross St. Martin 6**
**D-5000 Köln 1(DE)**

(54) Orthopädisches Verbandmaterial mit verringerter Klebrigkeit und vermindertem Rutschverhalten.

(57) Orthopädischer Steifverband, bei dem ein textiles Flächengebilde, welches mit einem reaktiven, flüssigen Polyisocyanatprepolymeren, das bei Befeuchtung mit Wasser härtet, imprägniert und/oder beschichtet ist, mit einer Mischung aus Öl und einem polymeren Mikropulver behandelt wird, um ein Material herzustellen, das reduzierte Klebrigkeit, reduziertes Rutschverhalten und reduzierte Schaumeigenschaften im Vergleich zu ähnlichen handelsüblichen Produkten aufweist. Ferner ist das Material vorzugsweise mit einem Füllmaterial, wie amorphem Silikat, beschichtet oder imprägniert.

EP 0 355 635 A1

# Orthopädisches Verbandmaterial mit verringerter Klebrigkeit und vermindertem Rutschverhalten

Die vorliegende Erfindung betrifft orthopädisches Verbandmaterial. Die Erfindung betrifft insbesondere solche Verbandmaterialien, die textile Flächengebilde umfassen, die mit Polyisocyanatprepolymeren imprägniert und/oder beschichtet sind, und die mit Mineralöl und Fluorkohlenstoffpolymerpulver behandelt werden, um die Klebrigkeit, das Rutschen und die Schäumeigenschaften des Materials während der Aushärtung zu reduzieren.

Die meisten heute zur Verfügung stehenden orthopädischen Steifverbände werden unter Verwendung aushärtbarer Harze hergestellt, die auf ein Substrat wie Glasfaser, Polyester oder andere synthetische oder natürliche textile Flächengebilde aufgebracht werden. So sind z.B. orthopädische Steifverbände mit Polyisocyanatprepolymeren, die mit Wasser reagieren und so die Aushärtung zu Polyurethanen initiieren, bereits bekannt (US-PS 4,411,262 Bonin et al, US-PS 4,502,479 Garwood et al). Im allgemeinen umfaßt das Polyisocyanatprepolymer das Reaktionsprodukt eines Isocyanats und eines Polyols, welches bei Kontakt mit Wasser zu Polyurethan polymerisiert. Die mit Prepolymer behandelte Binde wird vor dem Aufbringen auf ein Körperteil mit Wasser getränkt, und die nasse Binde wird dann auf den Körperteil aufgebracht. Nach Aufbringen der Binde wird der Steifverband mit einem Handschuh geglättet und an bestimmten Punkten festgehalten, bis er hart wird. Da die Harze in dem Verband bis zur Aushärtung sehr klebrig sind, bleiben die Schutzhandschuhe der Person, die den Verband anbringt, oftmals am Verband kleben. Dies ist von Nachteil, da es zum Aufwickeln des Verbandes kommen kann, da Schichten des Verbandes sich voneinander trennen, und der Verband somit nicht mehr geformt werden kann.

Um die "Klebrigkeit" aushärtbarer, harzbeschichteter Verbände zur verringern, wurde von Scholz et al in US-PS 4,667,661 vorgeschlagen, solche Verbandmaterialien mit bestimmten Schmiermitteln zu behandeln, um den kinetischen Reibungskoeffizienten solcher Flächen auf weniger als etwa 1,2 zu reduzieren. Dieses Gleitmittel kann aus (a) hydrophilen Gruppen, die kovalent an das aushärtbare Harz gebunden sind, (b) einem Hilfsstoff, der mit dem aushärtbaren Harz inkompatibel ist oder (c) einer Kombination aus (a) und (b) bestehen. Wie Scholz et al weiter ausführen (z.B. Spalte 11, Zeilen 21 ff), werden mit solchen Gleitmitteln behandelte Verbände sehr rutschig, und das leichte Verformen des Verbandes wird erleichtert wegen der nichtklebrigen Eigenschaften des Harzes. Im gleichen Patent (Spalte 8, Zeilen 45-65) wird ferner angeführt, daß Materialien wie Mineralöl als Schmiermittel beurteilt wurden, und, obwohl sie der Oberfläche des Verbandes eine nichtklebrige und sogar rutschige Eigenschaft verliehen, die einfache Anbringung und Formbarkeit der Binde am Patienten erlaubt, diese Wirkung nur vorübergehender Natur war. Im Durchschnitt hielten diese Materialien nur von einem Tag bis zu einer Woche, augenscheinlich wegen der Auflösung des Öls in dem Harz.

Es wird angenommen, daß Produkte nach dem Scholz et al Patent im Handel bereits erhältlich sind. Die Anwender beklagen aber oft, daß diese Verbandmaterialien, obwohl sie durchaus nicht so klebrig wie andere, frühere Binden sind, in der Tat zu schlüpfrig oder rutschig sind, um eine einfache Handhabung und Anbringung zu ermöglichen.

Ein weiteres Problem im Zusammenhang mit Flächenmaterialien, die mit Polyurethanprepolymerharz beschichtet oder impräg niert sind, ist das Schäumen des Harzes. Das Schäumen tritt auf, wenn Kohlendioxid durch Reaktion von Wasser mit Isocyanatgruppen im Harz freigesetzt wird. Wegen der Verringerung der Porosität und der Festigkeit des Verbandmaterials ist die Schaumbildung unerwünscht. Es wurde vorgeschlagen (US-PS 4,667,661), zur Verringerung der Schaumbildung dem Harz Schaumdämpfungsmittel, wie Silikon Antifoam A (Dow Corning) oder DB-100 Silikonfluid (Dow Corning), zuzugeben, doch der Einsatz sehr hydrophiler Hilfsstoffe, wie sie von Scholz et al zur Herstellung nichtklebriger Verbände vorgeschlagen werden, verstärkt das Problem der Schaumbildung im Verband.

Es besteht daher ein Bedarf an Verbandmaterialien mit verbesserten Handhabungseigenschaften, d.h. die weder zu klebrig noch zu rutschig sind, und die nicht so stark schäumen wie die handelsüblichen Produkte.

Es wurde nun gefunden, daß solche Verbände durch Beschichten oder Imprägnieren einer wasserhärtbaren Harzbahn mit einer Mischung aus Öl und polymerem Mikropulver hergestellt werden können. Daher betrifft die vorliegende Erfindung einen orthopädischen Steifverband, enthaltend ein textiles Flächengebilde, das imprägniert und/oder beschichtet ist mit einer Kombination eines reaktiven flüssigen Polyisocyanatprepolymeren, das bei Befeuchtung des Harzes mit Wasser aushärtet, und einer Mischung aus einem Öl, welches mit dem Prepolymeren nicht mischbar ist, und polymerem Mikropulver. Vorzugsweise sind die Bahnen zudem mit einer geringen Menge an anorganischem Füllmaterial mit hohem Volumen und geringer Dichte beschichtet oder imprägniert. Die Verbandmaterialien gemäß der vorliegenden Erfindung haben den Vorteil, daß sie leicht zu handhaben sind, d.h. sie sind weder zu klebrig noch zu rutschig. Ein zusätzlicher

2

EP 0 355 635 A1

Vorteil der erfindungsgemäß behandelten Bahnen ist, daß sie beim Aushärten und Härten eine sehr glatte Oberfläche aufweisen, die ästhetisch ansprechend ist und über die Kleidungsstücke leicht gleiten.

Textile Flächengebilde, die mit einem aushärtbaren Polyisocyanatprepolymer beschichtet sind oder in die ein solches Prepolymer imprägniert werden kann, sind im Stand der Technik ausführlich beschrieben (z.B. US-PS 4,667,661 und US-PS 4,411,262, deren Beschreibung durch Inbezugnahme in die vorliegende Anmeldung aufgenommen wird). Die Bahn ist halbstarr oder flexibel und sollte porös sein, so daß der Härter, Wasser, in die Rolle des textilen Flächenmaterials eindringen kann und alle Teile des Harzes berührt. Geeignete Bahnen sind z.B. Gewebe, Vliesstoffe oder Gewirke aus natürlichen oder synthetischen Fasern. Vorzugsweise sind die Bahnen Glasfasergewirke, es können jedoch auch textile Flächengebilde aus z.B. Baumwolle und Polyester eingesetzt werden.

Das erfindungsgemäß verwendete Polyisocyanatprepolymer enthält ein Prepolymer, das von Polyisocyanat abgeleitet und vorzugsweise aromatisch ist, und eine reaktive Wasserstoffverbindung oder ein Oligomer. Die bevorzugte Prepolymerzusammensetzung enthält modifiziertes Diphenylmethandiisocyanat, Polypropylenglykol, Benzoylchloridstabilisator und Dimorpholindiethyletherkatalysator. Das bevorzugte Polyisocyanat-Diolverhältnis ist etwa 4 zu 1 (NCO/OH = 4/1). Zur Verlängerung der Lagerbeständigkeit des Materials kann das Prepolymer bestimmte Stabilisatoren, wie Benzoylchlorid (0,1 bis 1,0 Gew.-%) enthalten, ebenso können Schaumdämpfungsmittel, wie Silikonflüssigkeiten enthalten sein.

Die auf das textile Flächengebilde aufgetragene Prepolymermenge muß ausreichen, um eine starke Laminatverbindung zwischen den Schichten zu bilden, darf jedoch nicht so groß sein, daß die Porosität unterbunden wird und der Harzfilm, der zur raschen und vollständigen Aushärtung möglichst dünn sein sollte, unnötig dick wird. Überschüssiges Prepolymer kann zu unsauberer Handhabung des textilen Flächengebildes infolge der Klebrigkeit oder von Tropfen und Abgabe von Harz führen. Das gewünschte Gewichtsverhältnis Harz/Trägermaterial ist eine Funktion sowohl der Viskosität des Prepolymeren als auch der Oberflächeneigenschaften des Trägermaterials und somit nur schwerlich genau quantifizierbar; der Fachmann wird jedoch ein geeignetes Verhältnis leicht bestimmen können.

Die vorteilhaften nichtklebrigen und doch nicht rutschigen Eigenschaften des erfindungsgemäßen Steifverbandes werden durch eine "Antiklebrigmacher"-Mischung erreicht, die ein Öl, das mit dem Prepolymeren nicht mischbar ist, und ein polymeres Mikropulver enthält, mit der das Verbandmaterial beschichtet wird und/oder die in das Verbandmaterial eingearbeitet wird. Es wird angenommen, daß das Pulver zur Immobilisierung des mineralischen Öls auf der Oberfläche des Verbandmaterials dient, so daß es weder freigesetzt wird und dadurch ein schmierig wirkendes Produkt erzeugt, noch von der Oberfläche wandert und dadurch für die Reduzierung der Klebrigkeit des aushärtenden Harzes nicht mehr zur Verfügung steht. Es wird ferner angenommen, daß die Anwesenheit der polymeren Mikropulver zu der glatten Oberfläche des ausgehärteten Verbandes beiträgt.

Mineralöl kommt erfindungsgemäß bevorzugt zur Anwendung, da es inert, ungiftig, stabil und homogen ist, und da es keinen unangenehmen Geruch aufweist; es können jedoch auch Öle wie Sojaöl, Maiskeimöl, Erdnußöl, Safloröl, Olivenöl, Sonnenblumenöl und Pflanzenöl verwendet werden.

Die erfindungsgemäß verwendeten Mikropulver weisen vorzugsweise eine Durchschnittsteilchengröße von nicht größer als etwa 10 Mikron auf, weiter bevorzugt von nicht größer als etwa 1 Mikron. Fluorkohlenstoffpolymerpulver sind besonders bevorzugt, da sie inert, stabil und ungiftig sind. Fluorkohlenstoffpolymerpulver sind in einer durchschnittlichen Teilchengröße in der Größenordnung von 50 Mikron erhältlich. Je größer die Teilchengröße, umso kleiner ist jedoch die Gesamtoberfläche der Pulver, und damit ist es umso wahrscheinlicher, daß das Pulver sich vom Öl trennt, was zu geringerer Immobilisierung des Öls führt. Die besten Ergebnisse wurden mit Fluorkohlenstoffpulver einer Durchschnittsteilchengröße von kleiner als etwa 1 Mikron erreicht. Andere polymere Mikropulver können anstelle von oder in Kombination mit Fluorkohlenstoffpulvern verwendet werden. Beispiele geeigneter Mikropulver sind Polyolefine, insbesondere Polyethylenmikropulver. Beispiele für im Handel erhältliche polymere Mikropulver, die für die vorliegende Erfindung geeignet sind, sind u.a. Slyp-Ayd Micronized Powder Polyolefin Blend der Firma Daniel Products Co., Jersey City, welches ein trockenes, mikronisiertes Pulver, einer Zusammensetzung aus halogenierten Polyolefinen, Polyethylenen und anderen synthetischen Wachsen darstellt; und Teflon DLX-6000 Industrial Fluoropolymer der Firma E.I. du Pont de Nemours and Co., Wilmington, Delaware.

In einer bevorzugten Ausführungsform der vorliegenden Erfindung wird in das Verbandmaterial auch ein anorganisches Füllmaterial mit hohem Volumen und geringer Dichte, wie z.B. amorphes Silikatpulver, eingearbeitet. Versuche zeigen, daß die Anwesenheit geringer Mengen dieses Füllmaterials (z.B. bis zu etwa 30 Gew.-%, vorzugsweise etwa 10 Gew.-%, bezogen auf die Antiklebrigmachermischung) die Aushärtzeit verringert und die Druckfestigkeit des Verbandmaterials verbessert. Das anorganische Füllmaterial weist vorzugsweise eine Durchschnittsteilchengröße in der Größenordnung von etwa 25 bis 30 Mikron auf. Es können auch andere anorganische Füllmaterialien, wie z.B. Glimmer verwendet werden, jedoch sind

3

amorphe Silikate wie Dicaperl (Fa. Grefco Inc., Torrance, Kanada) wegen ihrer Blasenform (hohle Kugel) und ihrer geringen Schüttdichte bevorzugt. Pyrogene Kieselsäuren sind ebenfalls bevorzugte Materialien. Das Füllmaterial kann mit dem Öl und dem polymeren Mikropulver eingemischt werden und, wie weiter unten beschrieben, auf den Verband gebracht werden. Eine andere Möglichkeit besteht darin, das amorphe Silikat mit dem Polyisocyanatprepolymeren einzumischen, das Prepolymer auf das Trägermaterial aufzutragen und dann mit der Öl.Pulver-Mischung zu beschichten.

Im allgemeinen enthält die Antiklebermischung 2-40% (Gew.-%) an polymerem Mikropulver, 60-98% Öl und 0-30% anorganisches Füllmaterial. Die besten Ergebnisse wurden mit einer Kombination aus 80 Gew.-% Mineralöl, 10 Gew.-% Fluorkohlenstoffpolymerpulver und 10 Gew.-% armorphem Silikat erzielt. Das Öl und das Fluorkohlenstoffpolymerpulver werden vorzugsweise mit hoher Scherkraft vermischt, bevor sie auf das Verbandmaterial gegeben werden. Die Mischung kann entweder auf die Oberfläche des Verbandmaterials aufgetragen werden, wie z.B. durch Beschichten oder Aufsprühen, oder in das Material imprägniert werden, wie z.B. durch Mischen mit dem Polyisocyanatprepolymeren vor Anwendung auf dem Trägermaterial.

Wird die Mineralöl/Pulvermischung als Beschichtung aufgetragen, geschieht dies vorzugsweise in einer Menge von etwa 2 bis etwa 10%, bezogen auf das Gewicht des Prepolymeren. Unterhalb von etwa 2% ist nicht genügend Mischung vorhanden, um die nichtklebenden und nichtrutschenden Eigenschaften zu erreichen, und oberhalb von etwa 10% ist das Produkt zu ölig. Die besten Ergebnisse wurden beim Beschichten oder Aufspritzen auf die Oberfläche mit einer Mischung aus 4 bis 5% der Öl/Pulvermischung, bezogen auf das Gewicht des Prepolymeren, erzielt. Die Mischung kann als vollflächige Beschichtung oder auch als unterbrochenes Muster, wie z.B. Streifen, aufgetragen werden. Wenn die Mineralöl/Pulvermischung in das Material imprägniert wird, so z.B. durch Mischen mit dem Polyisocyanatprepolymeren, wird eine größere Menge benötigt, um die gleichen nichtklebenden Eigenschaften zu erreichen. Im allgemeinen ist etwa 10 bis 20% der Mischung, bezogen auf das Gewicht des Prepolymeren, für diese Anwendungsform geeignet; die Anwesenheit der Mischung in dem Polyisocyanatprepolymeren kann jedoch auch nachteilig sein, dadurch, daß die Tendenz zur Verlangsamung der Aushärtreaktion besteht.

Die Materialien gemäß der vorliegenden Erfindung werden in den nachfolgenden Beispielen näher erläutert, ohne die Erfindung einzuschränken.

## BEISPIELE 1 - 8

## Gebundene Gleitmittel

## BEISPIEL 1

Zwei 3,8-1-Glasreaktionsgefäße (1 Gallone), jeweils ausgestattet mit einem 12,7 cm x 2,54 cm x 0,318 cm (5 x 1 x 1/8") Teflon (TM) Kreiselrührer, Zuführtrichter, Leitung zur Stickstoffspülung, Thermometer und einer Einlaßleitung für die Vakuumpumpe wurden montiert, und es wurde 30 Minuten gespült, um sicherzustellen, daß der gesamte Apparat vollständig trocken ist. Die Reaktionsgefäße wurden mit "A" und "B" gekennzeichnet.

## HARZVORMISCHUNG A

Die folgenden Chemikalien wurden nacheinander in 10-Minuten-Intervallen in das Reaktionsgefäß "A" gegeben:

4

| Chemikalie | Gew. (g) | Gew.-% |
|---|---|---|
| Isonate 143L (Upjohn Co.)[1] | 293,0 | 58,6 |
| SAG-47 (Union Carbide)[2] | 1,0 | 0,2 |
| Benzoylchlorid (Velsicol Chem.) | 0,5 | 0,1 |

[1]Diphenylmethandiisocyanat
[2]Silikon Antischaummittel

Das Gefäß wurde unter Rühren unter ein Vakuum von etwa -1 Atmosphäre gebracht und 1 Stunde getrocknet. Das Vakuum wurde entzogen, und der Rührer, das Thermometer und der Zuführtrichter wurden entfernt. Das Gefäß wurde dicht verschlossen.

HARZVORMISCHUNG B

Die folgenden Chemikalien wurden nacheinander in 10-Minuten-Intervallen in das Reaktionsgefäß "B" gegeben:

| Chemikalie | Gew. (g) | Gew.-% |
|---|---|---|
| Pluracol P 710 (BASF)[1] | 195,5 | 39,1 |
| DMDEE (Texaco Chemical)[2] | 10,0 | 2,0 |

[1]Polypropylenglykol
[2]Dimorpholindiethylether

Das Rühren wurde mit zunehmender Viskosität langsam verstärkt. Das Gefäß wurde unter Rühren unter Vakuum von etwa -1 Atmosphäre gebracht und 4 Stunden getrocknet.

Das Vakuum wurde entzogen, und der Rührer, das Thermometer und der Zuführtrichter wurden entfernt. Das Gefäß wurde dicht verschlossen.

HARZ: ZUGABE VON VORMISCHUNG "B" ZU VORMISCHUNG "A"

Beide Gefäße wurden unter kontinuierliche Stickstoffspülung gesetzt, und der Inhalt aus Gefäß "B" wurde zu dem Inhalt aus Gefäß "A" mit einer Geschwindigkeit von etwa 10,5 g pro Minute gegeben. Die Temperatur des Gefäßes stieg auf 55°C. Das Gefäß wurde kontinuierlich mit Stickstoff gespült und 1 1/2 Stunden gerührt.

ANTIKLEBRIGMACHER

Ein 3,8-1 Glasreaktionsgefäß, ausgestattet mit einem 12,7 cm x 2,54 cm x 0,318 cm Teflon (TM) Kreiselrührer, Zuführtrichter und Leitung zur Stickstoffspülung wurde montiert, und es wurde 30 Minuten gespült um sicherzustellen, daß der gesamte Apparat vollständig trocken ist. Die folgenden Chemikalien wurden durch den Zuführtrichter nacheinander in 5-Minuten-Intervallen in das Reaktionsgefäß gegeben.

| Chemikalie | Gew. (g) | Gew.-% |
|---|---|---|
| Drakeol 35 (Penreco)[1] | 80,0 | 80,0 |
| DLX6000 (DuPont)[2] | 10,0 | 10,0 |
| Dicaperl HP220[3] | 10,0 | 10,0 |

[1]Mineralöl
[2]Fluorkohlenstoffpolymermikropulver
[3]Amorphes Silikat

Mit zunehmender Viskosität wurde das Rühren langsam verstärkt. Das Gefäß wurde dicht verschlossen.

Der Inhalt des Gefäßes wurde unter kontinuierlicher Stickstoffspülung zu dem Inhalt aus Gefäß "A" gegeben. Gefäß "A" wurde 1 Stunde gerührt.

Ein 7,62 cm breites "Raschel-Knit" Glasfaserträgermaterial. bestehend aus 100% Owens-Corning ECDE 75 1/0 0,72 TPI Natural Color Glasfaserendlosgarn (vordere und rückseitige "bar"), wurde mit dem Harz beschichtet. Die Beschichtung aus Harz und Antiklebrigmacher betrug 48,5 Gew.-%. Das Material wurde zu Rollen von 3,66 m Länge verarbeitet. Diese Rollen wurden einzeln in feuchtigkeitsdichte Beutel verpackt.

Proben von 60,96 cm wurden nach einem und nach 10 Tagen gemäß der modifizierten ASTM D 1894 Methode, wie in der US-PS 4,667,661 vom 26.05.1987 beschrieben, geprüft. Die nach einem Tag geprüften Proben wiesen einen durchschnittlichen kinetischen Reibungskoeffizienten von 1,06 auf. Die nach 10 Tagen geprüften Proben wiesen ebenfalls einen mittleren kinetischen Reibungskoeffizienten von 1,06 auf.

Zudem wurde das Material auf Handhabungseigenschaften von einer Gruppe aus sechs Orthopädiemechanikern geprüft. Vor dem Wickeln der Testrollen wurde jeder Mechaniker gebeten, einige Rollen Scotchcast Plus der Firma 3M, Delta-Lite 'S' der Firma Johnson & Johnson und K-Cast der Firma Kirschner zu wickeln. Die Spezialisten wurden gebeten, das Testmaterial mit den o.g., handelsüblichen Produkten zu vergleichen. Jeder wurde hierzu um Stellungnahme gebeten.

Jedes Gruppenmitglied öffnete den Beutel, entnahm die Rolle (wobei OP-Handschuhe aus Vinyl getragen wurden) und tauchte sofort die Rolle 3 Sekunden lang in 23,9° C warmes Wasser. Dabei wurde die Rolle beim Untertauchen kräftig gedrückt, damit die Wasserverteilung in der Binde unterstützt wurde. Die Rolle wurde danach aus dem Wasser genommen und leicht gedrückt, um überschüssiges Wasser zu entfernen. Jeder Orthopädiemechaniker wickelte sodann die Rolle um einen 2" (5 cm) rostfreien Stahldorn, um einen Zylinder von etwa 8" (20 cm) Länge zu bilden. Die Handhabungseigenschaften wurden nun von den Mitgliedern der Gruppe bewertet nach: Abwickeln der Rolle, Formbarkeit, Wasserverlust, Schäumen und Aussehen nach Konfektionierung.

Die Handhabungseigenschaften wurden als sehr gut bezeichnet. Die Gutachter meinten, das Produkt schäume nicht und weise wesentlich geringeren Wasserverlust/Tropfen auf, als Scotchcast Plus von 3M und Delta-Lite S von Johnson & Johnson.

## BEISPIEL 2

Folgendes Harz und Antiklebrigmacher wurden hergestellt, vermischt, beschichtet und gemäß Beispiel 1 verpackt:

| Chemikalie | Gew. (g) | Gew.-% |
|---|---|---|
| Drakeol 35 (Penreco) | 60,0 | 80,0 |
| Acumist (TM) B-6 (Allied Signal)[1] | 7,5 | 10,0 |
| Dicaperl HP220 | 7,5 | 10,0 |

[1]Mikronisiertes Polyethylenwachs

Das Material wurde gemäß der KCOF-Methode geprüft und wies nach einem Tag einen durchschnittlichen kinetischen Reibungskoeffizienten von 1,1 und nach 10 Tagen von 1,09 auf.

Die Handhabungseigenschaften des Materials wurden als sehr gut bezeichnet. Kein Schäumen, und sehr sauber/kein Schmutz im Vergleich zu den oben genannten Produkten von 3M und Johnson & Johnson.

## BEISPIEL 3

Folgendes Harz und Antiklebrigmacher wurden hergestellt, vermischt, beschichtet und gemäß Beispiel 1 verpackt:

| Chemikalie | Gew. (g) | Gew.-% |
|---|---|---|
| Sojaöl | 80,0 | 80,0 |
| DLX6000 | 10,0 | 10,0 |
| Dicaperl HP220 | 10,0 | 10,0 |

Das Material wurde gemäß der KCOF-Methode geprüft und wies nach einem Tag einen durchschnittlichen kinetischen Reibungskoeffizienten von 1,08 und nach 10 Tagen von 1,09 auf.

Die Handhabungseigenschaften des Materials wurden als sehr gut bezeichnet. Kein Schäumen, und sehr sauber/kein Schmutz im Vergleich zu den oben genannten Produkten von 3M und Johnson & Johnson.

## BEISPIEL 4

Folgendes Harz und Antikleber wurden hergestellt, vermischt, beschichtet und gemäß Beispiel 1 verpackt:

| Chemikalie | Gew. (g) | Gew.-% |
|---|---|---|
| Maiskeimöl | 40,0 | 80,0 |
| Acumist (TM) B-6 | 5,0 | 10,0 |
| Dicaperl HP520 | 5,0 | 10,0 |

Das Material wurde gemäß der KCOF-Methode geprüft und wies nach einem Tag einen durchschnittlichen kinetischen Reibungskoeffizienten von 1,32 und nach 10 Tagen von 1,32 auf.

Das Expertenteam beurteilte das Material als sauber und glatt. Es wurde angegeben, daß das Material zu trocken bzw. klebrig sei. Augenfällig war ein erheblicher Widerstand gegen Verformung. Kein Schäumen, und sehr sauber/kein Schmutz im Vergleich zu den oben genannten Produkten von 3M und Johnson & Johnson.

## BEISPIEL 5

Folgendes Harz und Antiklebrigmacher wurden hergestellt, vermischt, beschichtet und gemäß Beispiel 1 verpackt:

| Chemikalie | Gew. (g) | Gew.-% |
|---|---|---|
| Erdnußöl | 89,0 | 80,0 |
| Acumist (TM) B-6 | 10,0 | 10,0 |
| Cab-o-sil[1] | 1,0 | 1,0 |

[1]pyrogene Kieselsäure

Das Material wurde gemäß der KCOF-Methode geprüft und wies nach einem Tag einen durchschnittlichen kinetischen Reibungskoeffizienten von 1,22 und nach 10 Tagen von 1,22 auf.

Die Handhabungseigenschaften des Materials wurden als ausgezeichnet bezeichnet und als den handelsüblichen Produkten überlegen.

BEISPIEL 6

Folgendes Harz und Antiklebrigmacher wurden hergestellt, vermischt, beschichtet und gemäß Beispiel 1 verpackt:

| Chemikalie | Gew. (g) | Gew.-% |
|---|---|---|
| Safloröl | 60,0 | 80,0 |
| DLX6000 | 7,5 | 10,0 |
| Dicaperl HP220 | 7,5 | 10,0 |

Das Material wurde gemäß der KCOF-Methode geprüft und wies nach einem Tag einen durchschnittlichen kinetischen Reibungskoeffizienten von 1,24 und nach 10 Tagen von 1,25 auf.

Die Handhabungseigenschaften des Materials wurden als ausgezeichnet bezeichnet und als den handelsüblichen überlegen.

BEISPIEL 7

Folgendes Harz und Antiklebrigmacher wurden hergestellt, vermischt, beschichtet und gemäß Beispiel 1 verpackt:

| Chemikalie | Gew. (g) | Gew.-% |
|---|---|---|
| Olivenöl | 40,0 | 80,0 |
| DLX6000 | 5,0 | 10,0 |
| Aerosil R972[1] | 0,25 | 0,5 |

[1]pyrogene Kieselsäure

Das Material wurde gemäß der KCOF-Methode geprüft und wies nach einem Tag einen durchschnittlichen kinetischen Reibungskoeffizienten von 1,30 und nach 10 Tagen von 1,29 auf.

Die Gruppenmitglieder bezeichneten das Material als sehr gut, jedoch als fast zu trocken oder klebrig.

8

BEISPIEL 8

Folgendes Harz und Antiklebrigmacher wurden hergestellt, vermischt, beschichtet und gemäß Beispiel 1 verpackt:

| Chemikalie | Gew. (g) | Gew.-% |
|---|---|---|
| Sonnenblumenöl | 80,0 | 80,0 |
| Acumist (TM) B-6 | 10,0 | 10,0 |
| Dicaperl HP220 | 10,0 | 10,0 |

Das Material wurde gemäß der KCOF-Methode geprüft und wies nach einem Tag einen durchschnittli-- chen kinetischen Reibungskoeffizienten von 1,1 und nach 10 Tagen von 1,11 auf.

Die Handhabungseigenschaften des Materials wurden als sehr gut bezeichnet.

BEISPIELE 9-18

Oberflächenschmiermittel

BEISPIEL 9

Ein 3,8-1-Glasgefäß, ausgestattet mit einem 12,7 cm x 2,54 cm x 0,318 cm Teflon (*TM) Kreiselrührer, Zuführtrichter und Leitung zur Stickstoffspülung wurde montiert, und es wurde 30 Minuten gespült, um sicherzustellen, daß der gesamte Apparat vollständig trocken ist. Die folgenden Chemikalien wurden durch den Zuführtrichter nacheinander in 5-Minuten-Intervallen in das Reaktionsgefäß gegeben.

| Chemikalie | Gew. (g) | Gew.-% |
|---|---|---|
| Drakeol 35 (Penreco) | 480,0 | 80,0 |
| DLX6000 | 60,0 | 10,0 |
| Dicaperl HP220 | 60,0 | 10,0 |

Mit zunehmender Viskosität wurde das Rühren langsam verstärkt.

Der Antiklebrigmacher wurde auf ein 7,62 cm breites harzimprägniertes Glasfaserträgermaterial, das einen Harzanteil von 48,5 Gew.-% aufweist, beschichtet. Zum Aufbringen des Antiklebers in Streifen, die längs des Substrats verlaufen, wurde ein Profil-Förderwalzen-System angewandt. Die Streifen waren 1/8" (0,318 cm) breit und in 1/8" (0,318 cm) Intervallen angebracht. Diese Methode ist der vollflächigen Beschich tung vorzuziehen. Dieses Verfahren minimiert den Anteil an Antiklebrigmacher, der dem Substrat zugeführt werden muß, ohne die Handhabungseigenschaften in Mitleidenschaft zu ziehen.

Das beschichtete Trägermaterial wurde in einzelne Rollen von 3,66 m Länge überführt. Durch die Zugabe von Antiklebrigmacher zur Rolle erhöhte sich das Gewicht der Rolle um 5,2%. Diese beschichteten Rollen wurden einzeln in feuchtigkeitsdichte Beutel verpackt. 10 Tage nach dem Beschichten wurden 5 Rollen gemäß der modifizierten ASTM D 1894 Methode, wie in der US-PS 4,667,661 vom 26.05.87 beschrieben, geprüft. Der durchschnittliche kinetische Reibungskoeffizient lag nach einem Tag bei 1,28 und nach 10 Tagen bei 1,28.

Die Gruppe der orthopädischen Fachleute beurteilte die Handhabungseigenschaften als sehr gut.

BEISPIEL 10

Folgender Antiklebrigmacher wurde zubereitet, aufgebracht und gemäß dem Verfahren nach Beispiel 9 verpackt:

| Chemikalie | Gew. (g) | Gew.-% |
|---|---|---|
| Drakeol 35 | 480,0 | 80,0 |
| Slip-Ayd - SL 600[1] | 60,0 | 10,0 |
| Dicaperl HP220 | 60,0 | 10,0 |

[1]Mikronisierte Polyolefinpulvermischung

Das Material wurde gemäß der KCOF-Methode geprüft und wies einen durchschnittlichen kinetischen Reibungskoeffizienten von 1,25 auf.

Die Handhabungseigenschaften des Materials wurden von dem orthopädischen Expertenteam als ausgezeichnet beurteilt.

BEISPIEL 11

Der Antikleber wurde vorbereitet, aufgebracht, und das Material wurde gemäß Beispiel 9 verpackt:

| Chemikalie | Gew. (g) | Gew.-% |
|---|---|---|
| Sojaöl | 480,0 | 80,0 |
| DLX6000 | 60,0 | 10,0 |
| Dicaperl HP220 | 60,0 | 10,0 |

Das Material wurde gemäß der KCOF-Methode geprüft und wies nach einem Tag einen durchschnittlichen kinetischen Reibungskoeffizienten von 1,22 und nach 10 Tagen von 1,23 auf.

Die Handhabungseigenschaften des Materials wurden als ausgezeichnet bezeichnet.

BEISPIEL 12

Der folgende Antiklebrigmacher wurde gemäß Beispiel 1 vorgelegt. Eine profilierte Förderwalze wurde eingesetzt, um den Antiklebrigmacher in 1/8" Streifen (die Länge des Bandes entlang) in 3/16" Intervallen aufzubringen.

| Chemikalie | Gew. (g) | Gew.-% |
|---|---|---|
| Maiskeimöl | 480,0 | 80,0 |
| Acumist (TM) B-6 | 60,0 | 10,0 |
| Dicaperl HP520 | 60,0 | 10,0 |

Das Material wurde gemäß der KCOF-Methode geprüft und wies nach einem Tag einen durchschnittlichen kinetischen Reibungskoeffizienten von 1,57 und nach 10 Tagen von 1,58 auf.

Die Mitglieder des Expertenteams beurteilten das Material als schlecht. Das Material sei zu trocken oder klebrig. Augenfällig war der erhebliche Widerstand gegenüber Verformbarkeit.

Das Team merkte an, daß dieses Material weniger klebrig und eher akzeptabel war als K-Cast der Firma Kirschner.

BEISPIEL 13

Folgender Antiklebrigmacher wurde hergestellt, aufgebracht und gemäß Beispiel 9 verpackt:

| Chemikalie | Gew. (g) | Gew.-% |
|---|---|---|
| Erdnußöl | 537,0 | 89,5 |
| Acumist (TM) B-6 | 60,0 | 10,0 |
| Cab-o-sil | 3,0 | 0,5 |

Das Material wurde gemäß der KCOF-Methode geprüft und wies nach einem Tag einen durchschnittlichen kinetischen Reibungskoeffizienten von 1,17 und nach 10 Tagen von 1,17 auf.

Die Handhabungseigenschaften dieses Materials wurden als ausgezeichnet beurteilt.

BEISPIEL 14

Folgender Antikleber wurde hergestellt, aufgebracht und gemäß Beispiel 9 verpackt:

| Chemikalie | Gew. (g) | Gew.-% |
|---|---|---|
| Safloröl | 480,0 | 80,0 |
| DLX6000 | 60,0 | 10,0 |
| Dicaperl HP220 | 60,0 | 10,0 |

Das Material wurde gemäß der KCOF-Methode geprüft und wies nach einem Tag einen durchschnittlichen kinetischen Reibungskoeffizienten von 1,28 und nach 10 Tagen von 1,29 auf.

Die Handhabungseigenschaften dieses Materials wurden als sehr gut beurteilt.

BEISPIEL 15

Folgender Antiklebrigmacher wurde hergestellt, aufgebracht und gemäß Beispiel 9 verpackt:

| Chemikalie | Gew. (g) | Gew.-% |
|---|---|---|
| Olivenöl | 534,0 | 89,0 |
| DLX6000 | 60,0 | 10,0 |
| Aerosil R972 | 6,0 | 1,0 |

11

Das Material wurde gemäß der KCOF-Methode geprüft und wies nach einem Tag einen durchschnittlichen kinetischen Reibungskoeffizienten von 1,35 und nach 10 Tagen von 1,35 auf.

Das Expertenteam beurteilte dieses Materials als gut, jedoch als fast zu trocken bzw. klebrig.

BEISPIEL 16

Der folgende Antiklebrigmacher wurde hergestellt, aufgebracht und gemäß Beispiel 9 verpackt:

| Chemikalie | Gew. (g) | Gew.-% |
|---|---|---|
| Sonnenblumenöl | 480,0 | 80,0 |
| Acumist (TM) B-6 | 60,0 | 10,0 |
| Dicaperl HP820 | 60,0 | 10,0 |

Das Material wurde gemäß der KCOF-Methode geprüft und wies nach einem Tag einen durchschnittlichen kinetischen Reibungskoeffizienten von 1,1 und nach 10 Tagen von 1,11 auf.

Die Handhabungseigenschaften dieses Materials wurden als sehr gut beurteilt.

BEISPIEL 17

Folgender Antikleber wurde hergestellt, aufgebracht und gemäß Beispiel 9 verpackt:

| Chemikalie | Gew. (g) | Gew.-% |
|---|---|---|
| Drakeol 35 | 480,0 | 80,0 |
| Acumist (TM) B-6 | 120,0 | 10,0 |

Das Material wurde gemäß der KCOF-Methode geprüft und wies nach einem Tag einen durchschnittlichen kinetischen Reibungskoeffizienten von 0,51 und nach 10 Tagen von 0,62 auf.

Das Material wurde als zu rutschig und nicht so gut handhabbar bzw. trocken verglichen mit den vorhergehenden Testbei spielen beurteilt. Das Expertenteam merkte an, daß dieses Material in Bezug auf die Handhabung dem Scotchcast Plus von 3M und Delta-Lite 'S' von Johnson & Johnson nahezu gleichwertig war. Das vorliegende Material wurde als glatt und sauber beurteilt. Die Gruppenmitglieder beurteilten es als schwierig, die Rolle festzuhalten während das Band gezogen wurde (das Band wurde gezogen, um die Vorgehensweisen beim Anbringen des Bandes auf Gliedmassen des Patienten nachzuempfinden).

BEISPIEL 18

Unbehandelter Steifverband

Gemäß Beispiel 1 vorbereitetes und beschichtetes Harz wurde nach der KCOF-Methode geprüft und wies einen durchschnittlichen kinetischen Reibungskoeffizienten von 2,56 nach einem Tag und von 2,56 nach 10 Tagen auf.

Es wurde als sehr schwierig beurteilt, mit diesem Material zu arbeiten. Die Experten beanstandeten dieses Material aufgrund seiner extremen Klebrigkeit.

Ansprüche

1. Orthopädischer Steifverband, enthaltend ein textiles Flächengebilde, welches mit einer Kombination aus einem reaktiven, flüssigen Polyisocyanatprepolymeren, das bei Befeuchtung des Harzes mit Wasser härtet, und einer Antiklebrigmachermischung beschichtet oder imprägniert ist, die ein mit dem genannten Prepolymeren nicht mischbares Öl und ein polymeres Mikropulver enthält.

2. Steifverband gemäß Anspruch 1, bei dem das textile Flächengebilde ein Glasfasergewirke ist.

3. Steifverband gemäß Anspruch 1, bei dem das Polyisocyanatprepolymer das Reaktionsprodukt aus Diphenylmethandiisocyanat und Polypropylenglykol enthält.

4. Steifverband gemäß Anspruch 1, bei dem das Öl Mineralöl ist.

5. Steifverband gemäß Anspruch 1, bei dem das polymere Mikropulver eine Durchschnittsteilchengröße von nicht mehr als etwa 10 Mikron aufweist.

6. Steifverband gemäß Anspruch 1, bei dem das polymere Mikropulver aus Fluorkohlenstoffpolymerpulvern, Polyolefinpulvern und Mischungen daraus ausgewählt ist.

7. Steifverband gemäß Anspruch 1, bei dem das polymere Mikropulver ein Fluorkohlenstoffpolymerpulver mit einer Durchschnittsteilchengröße von nicht mehr als etwa 1 Mikron ist.

8. Steifverband gemäß Anspruch 7, bei dem das Öl Mineralöl ist.

9. Steifverband gemäß Anspruch 1, welches ferner ein großvolumiges anorganisches Füllmaterial geringer Dichte enthält.

10. Steifverband gemäß Anspruch 9, bei dem das Füllmaterial aus amorphen Silikaten und pyrogenen Kieselsäuren ausgewählt wird.

11. Steifverband gemäß Anspruch 10, bei dem das Füllmaterial eine Durchschnittsteilchengröße in dem Bereich von etwa 25 bis 30 Mikron aufweist.

12. Steifverband gemäß Anspruch 1, bei dem die Antiklebrigmachermischung etwa 2 bis 40 Gew.-% polymeres Mikropulver, etwa 60 bis 98 Gew.-% Öl und 0 bis 30 Gew.-% anorganisches Füllmaterial enthält.

13. Steifverband gemäß Anspruch 12, bei dem das polymere Mikropulver aus Fluorkohlenstoffpolymerpulvern und Polyolefinpulvern und Mischungen daraus ausgewählt wird, das Öl Mineralöl ist und das anorganische Füllmaterial aus amorphen Silikaten und pyrogenen Kieselsäuren ausgewählt wird.

14. Steifverband gemäß Anspruch 12, bei dem das polymere Mikropulver Fluorkohlenstoffpolymerpulver mit einer Durchschnittsteilchengröße von nicht mehr als etwa 1 Mikron ist und das anorganische Füllmaterial eine Durchschnittsteilchengröße von etwa 25 bis 30 Mikron aufweist.

15. Steifverband gemäß Anspruch 1, bei dem die Antiklebrigmachermischung auf das textile Flächengebilde als Beschichtung in einem Anteil von etwa 2 bis 10%, bezogen auf das Gewicht des Prepolymeren, aufgetragen wird.

16. Steifverband gemäß Anspruch 15, bei dem die Antiklebrigmachermischung als Beschichtung in einem Anteil von etwa 4 bis 5%, bezogen auf das Gewicht des Prepolymeren, aufgetragen wird.

17. Steifverband gemäß Anspruch 1, bei dem vor Applikation auf das textile Flächengebilde die Antiklebrigmachermischung mit dem Prepolymeren kombiniert wird.

## EUROPÄISCHER RECHERCHENBERICHT

Europäisches Patentamt

Nummer der Anmeldung

EP 89 11 4949

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 259 492  (A.B. DAVYDOV) <br> * Seite 3, Zeilen 32-34; Ansprüche 1,2 * <br> ----- | 1 | A 61 L 15/07 |

| | | | RECHERCHIERTE SACHGEBIETE (Int. Cl.5) |
|---|---|---|---|
| | | | A 61 L |

Der vorliegende Recherchenbericht wurde für alle Patentansprüche erstellt

| Recherchenort | Abschlußdatum der Recherche | Prüfer |
|---|---|---|
| DEN HAAG | 13-10-1989 | PELTRE CHR. |